# EUROPEAN PATENT SPECIFICATION

(11) **EP 0 781 089 B1**
(45) Date of publication and mention of the grant of the patent: **24.11.2004**
(21) Application number: 95934975.4
(22) Date of filing: 18.09.1995
(51) Int. Cl.: A61F 2/06, A61F 2/24, A61L 33/00

(54) **THROMBORESISTANT SURFACE TREATMENT FOR BIOMATERIALS**
THROMBOSERESISTENTE OBERFLÄCHENBEHANDLUNG VON BIOMATERIALIEN
TRAITEMENT DE SURFACE THROMBORESISTANT POUR BIOMATERIAUX

(30) Priority: 16.09.1994 US 307335
(43) Date of publication of application: 02.07.1997
(73) Proprietor: Haimovich, Beatrice, North Brunswick, NJ 08902 (US); Freeman, Amihay, 73112 Israel (IL); Greco, Ralph, Warren, NJ 07059 (US)
(72) Inventor: Haimovich, Beatrice, North Brunswick, NJ 08902 (US); Freeman, Amihay, 73112 Israel (IL); Greco, Ralph, Warren, NJ 07059 (US)
(74) Representative: Pernez, Helga
(86) International application number: PCT/US1995/011646
(87) International publication number: WO 1996/008149

(56) References cited:
- US-A- 3 962 158
- US-A- 4 321 711
- US-A- 4 326 532
- US-A- 4 787 900
- US-A- 4 822 361

## Description

### Field of the Invention

The present invention generally relates to medical devices having a blood contacting surface which has thromboresistant properties, more particularly, the invention relates to the surface treatment of medical devices with a thromboresistant coating on the surface which is intended for contacting blood and other body fluids where said coating is composed of a chitosan-based membrane incorporating thromboresistant components affecting surface properties.

### Description of Related Art

A well-recognized problem in the medical community is the development of a thrombus or blood clot or obstruction that forms when any number of devices are used either within the body or within systems wherein blood or other body fluids are contacted or circulated. Such devices include, for example, catheters, vascular grafts, cardiac pacemaker leads, replacement heart valves, sutures, angioplasty devices, and various blood diagnostic, treatment, and storage systems and may be composed of a variety of biomaterials, for example, metals, polymers, and rubbers.

Of special interest are the approximately 350,000 small vessel bypass grafts performed annually in the United States. The saphenous vein is the choice for small vessel replacement but involves many possible limitations and complications; for example, adequate saphenous vein may not be available due to disease or previous usage. Prosthetic grafts made of expanded polytetrafluoroethylene (ePTFE) or Dacron have been used quite successfully for large vessel replacement but almost uniformly fail when used in sizes of less than 6 mm internal diameters, Callow, A.D., Current Status of Vascular Grafts, Surg. Clin. No. Amer. **65**, 501 (1982). There is an urgent need for the development of a small vascular prosthesis (about 3-4 mm inside diameter) that exhibit thromboresistance for use in distal extremity bypasses, coronary artery bypasses, and a variety of other vascular access procedures.

The host response to biomaterials at the cellular and molecular level is complex. For example see, *Implantation Biology, The Host Response and Biomedical Devices,* (Greco, R.S. ed. 1994). The composition of the biomaterial surface appears to play an important role in thrombosis. Thrombosis is initiated by the adsorption of plasma proteins on the biomaterial surface. This adsorption results from, and is dependent upon, the physical and chemical properties of the biomaterial surface. For example, Dacron has been shown to have a greater effect on platelet activation than ePTFE, while ePTFE is known to be a stronger stimulator of fibrous hyperplasia. Deposition of plasma proteins on the biomaterial surface mediates platelet adhesion via platelet surface receptors. Surface morphology and chemical characteristics, for example, smoothness and hydrophilicity, have been shown to enhance low plasma protein adsorptive properties. As a consequence of the decrease in plasma proteins deposited on the surface, less adhesive matrix proteins will be available to mediate platelet binding and activation on the surfaces, thus leading to reduced surface-initiated thrombosis.

A variety of approaches have been attempted to increase the thromboresistance of these surfaces. These approaches include the bonding of heparin, albumin, or polyurethanes to the surface, the seeding of endothelial cells onto the surface, and the pretreatment of platelets with adhesion receptor specific monoclonal antibodies. All of these approaches, however, have had limited success.

The known practice of graft polymerization of monomers onto the substrate to form thromboresistant polymers on the surface of biomaterials has several drawbacks. Graft polymerization can affect the original chemical structure of the coated biomaterial and has the danger of toxic low molecular residues of the toxic monomers, as discussed in U.S. Patents No. 4,978,481 to Janssen et al. and 4,311,573 to Mayhan et al. The grafting process can be complicated and expensive, e.g., requiring elaborate chemical treatment of the substrate. For example, United States Patent No. 5,053,048 to Pinchuk teaches the pretreatment of a substrate before grafting a polymer; in U. S. Patent No. 4,978,481 the substrate is pre-treated with ozone before grafting the monomer; and in U. S. Patent No. 4,311,573 the substrate is pre-treated with ozonization or high energy ionizing radiation before the graft polymerization.

An alternative approach to graft polymerization which is based on absorption of biopolymers has been described. Malett, et al, *Chitosan: A New Hemostatic*, Annals of Thoracic Surgery, **36**, 55 (1983) examined the effect of soaking large diameter Dacron vascular grafts in an acidic chitosan solution, a deacetylated derivative of arthropod chitin, as related to the leakage of these grafts. Examination of these grafts at 24 hours revealed no rebleeding. The data establishes that chitosan has thrombogenic properties which induced clot formation rendering the vascular grafts impermeable to blood. Similar thrombogenic properties of chitosan were reported by van der Lei, et al., *Improved Healing of Microvascular PTFE Prostheses by Induction of a Clot Layer: An Experimental study in Rats*, Plastic and Reconstructive Surgery, **84**, 960 (1989). In their study ePTFE grafts soaked in a diluted acidic chitosan solution that were implanted into rats were covered with a clot layer, indicative of a highly thrombogenic surface. Both studies demonstrate the thrombogenic properties of chitosan as exhibited by the molecular adsorption of chitosan on the surface of the grafts employed. Neither study reported that the adsorbed chitosan solution formed a distinct coating layer. Nevertheless, a tubular prosthesis of porous PTFE with a coating of chitosan and heparin is disclosed in US patent No 4 822 361, while the US patents No. 4 787 900 and No. 4 326 532 disclose a blood vessel prosthesis with a coating layer of collagen and chitosan and a polymeric medical article coated with chitosan to which an antithrombogenic agent is bonded, respectively.

In contrast to chitosan's thrombogenic properties, polyvinylalcohol (PVA) has been reported to exhibit non-thrombogenic properties. Miyake et al., *New Small-Caliber Antithrombotic Vascular Prosthesis: Experimental Study*, Microsurgery, **5**, 144 (1984), reported that tubes made of polyvinylalcohol were non-thrombogenic. These tubes, however, were not suitable for anastomosis since they were not durable enough to withstand tearing by suture.

The US patent No. 3 962 158 describes a blend of PVA and chitosan useful as semipermeable membranes or ultrafilter membranes.

What is needed is a biomaterial with a non-thrombogenic surface suitable for use in vascular grafts having an inside diameter of less than 6 millimeters and an inexpensive, efficient means for providing consistent non-thrombogenic properties to the surface of the biomaterial.

The present invention provides a biomaterial with a thromboresistant coating, and a method for preparing same. The features and advantages of this invention will be clearly understood through a consideration of the following description.

### SUMMARY OF THE INVENTION

The present invention pertains to the surface treatment of biomaterials for use in medical devices which provides a non-thrombogenic surface as a result of a surface coating with a chitosan/polyvinyl alcohol based membrane. The construction of a non-thrombogenic chitosan-based membrane is affected by contacting the biomaterial surfaces with mixtures comprising acidic chitosan solutions, polyvinyl alcohol solutions, and non-ionic detergent. Following a stabilization treatment by air drying or cross-linking, the adsorbed film forms a stable membrane that affects both the morphology and the chemical characteristics of the biomaterial surface.

Coated biomaterials obtained using this process exhibit a smoother surface and strong structural morphology with improved chemical surface characteristics resulting in a significant decrease in platelet binding to the surface. This effect may be further improved by bioactive agents that may be embedded in the complex or bound to activated sites on the chitosan/polyvinyl alcohol membrane.

### BRIEF DESCRIPTION OF THE DRAWINGS

Figure 1 is a cross-sectional view of an illustration of a vascular graft with a thromboresistant surface of the present invention.
Figure 2 is a cross-sectional view of an Illustration of a vascular graft with another embodiment of the thromboresistant surface of the present invention.

### DETAILED DESCRIPTION OF THE INVENTION

The present invention produces medical devices which are composed of biomaterials having a thromboresistant surface. The thromboresistant properties are provided by a distinct coating layer applied to the surface which comprises a chitosan/ polyvinyl alcohol based membrane and biologically and/or chemically active materials. The active materials can be incorporated into the chitosan/polyvinyl alcohol based membrane and/or bound onto the surface of activated chitosan/polyvinyl alcohol in the coating layer.

The characteristics of the resulting coating and the method of forming the coating make possible thromboresistant medical devices which could not be manufactured with other coatings and/or methods. For example, vascular grafts with an inside diameter of less than 6 millimeters may now be effectively used and consistently manufactured having a thromboresistant surface according to the present invention.

The process for applying the thromboresistant coating to the biomaterial surface comprises the steps of:
1. Preparing a coating solution comprising a sterile aqueous solution of chitosan and polyvinyl alcohol with any necessary additives such as stabilizers, buffers, catalysts, and cross-linking agents.
2. The surface of the biomaterial is coated with the coating solution, for example, by pumping or by dipping; any excess solution is removed by draining.
3. The adsorbed solution is cured either by air or gas drying or by chemical cross-linking, using a cross-linking agent in an aqueous solution.
4. If desired, biologically active reagents, such as surfactants, antithrombotic agents, antibiotics or antibodies may be entrapped within the chitosan/polyvinyl alcohol based membrane during the initial coating formation or bonded and immobilized to its surface at activated sites on the chitosan after the initial coating formation.

The biomaterials which can be coated include polytetrafluoroethylenes, polyamides, polyesters, polyurethanes, polysiloxanes, polyolefins, Dacron, silicone rubber, polyvinyl chlorides, copolymers, metals, and the like. These biomaterials are commonly used in medical devices and procheses including catheters, vascular grafts, cardiac pacemakers, pacemaker leads, heart diaphragms, heart valves, sutures, needles, angioplasty devices, tubing, dialysis membranes, filters and other artificial implants and machines.

Chitosan is a natural polysaccharide which is soluble in acidic aqueous solutions (pH less than 5.5) and becomes insoluble at physiological pH, with a general structure resembling cellulose. Preferred chitosan concentrations range from about 0.5 - 3% (w/v). The construction of a chitosan/polyvinyl alcohol based membrane on certain biomaterials (for example, ePTFE) may require the incorporation of suitable additives, for example, a non-ionic detergent such as Triton X-100; without which, the chitosan/polyvinyl alcohol coating solution would be repelled from the ePTFE surface. Due to the presence of free amino groups on the chitosan, it may be readily chemically activated for the chemical binding of bioactive molecules.

Preferred polyvinyl alcohol solutions are comprised of about 1 - 5% (w/v) polyvinyl alcohol (PVA) having a molecular weight between about 10,000 to about 125,000. PVA containing coatings are preferably prepared with aqueous solutions and cured by air drying.

The coating is a complex of chitosan and polyvinyl alcohol (PVA). The PVA is incorporated into the chitosan-basad membrane forming a polymer blend. The coating solution for such a chitosan-PVA coating is prepared by adding a PVA solution to the chitosan solution with a chitosan:PVA ratio in the range of about 9:1 to about 1:1. The resulting coating layer unexpectedly exhibits essentially only the desirable qualities of the individual components. The chitosan provides a stable structural base for the incorporation of the PVA. The chitosan-PVA coating exhibits both the strong structural qualities of a chitosan membrane and the thromboresistant qualities of a PVA surface. These chitosan-PVA coatings exhibit none of the thrombogenic properties of chitosan and are significantly more strong and more stable than plain PVA coatings.

A preferred embodiment of a chitosan/PVA based coating is comprised of a chitosan/PVA based membrane and one or more bioactive materials, for example antithrombotic agents, antibiotics or antibodies, attached to the chitosan layer at sites which have been activated, for example by glutardialdehyde.

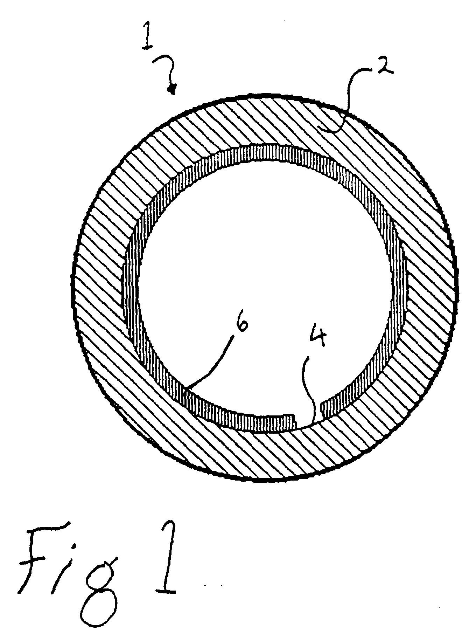

A preferred embodiment of a medical device incorporating the thromboresistant biomaterial is illustrated in figure 1 in the form of a vascular graft 1 used for small vessel bypass grafts comprising a section of vascular rubing 2, preferably made of Dacron or ePTFE, having an inside diameter of less than 6 mm, preferably less than 4 mm. On the inner surface of the tubing 4 is a thromboresistant coating 6 comprised of a chitosan-PVA blend.

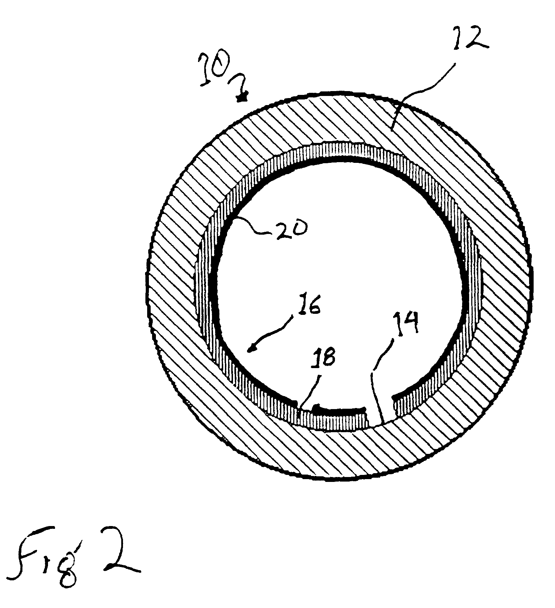

Another preferred embodiment of a medical device incorporating the thromboresistant biomaterial is illustrated in figure 2 in the form of a vascular graft 10 used for small vessel bypass grafts comprising a section of vascular tubing 12, preferably made of Dacron or ePTFE, having an inside diameter of less than 6 mm, preferably less than 4 mm. On the inner surface of the tubing 14 is a thromboresistant coating 16 comprised of a chitosan membrane 18 with a bonded layer of biologically active molecules 20.

The invention is further and more specifically illustrated by the following examples and tests.

### EXAMPLE 1

Biomaterials with a thromboresistant coating of PVA were prepared as follows:

### Preparation of PVA solution

A 5% (w/v) polymer solution was prepared by stirring 2.5 grams PVA (BDH, Cat. No. 2979) in 50 mLs of warm distilled water. Similar polymer solutions were prepared at concentrations of 0.5%, 1%, and 2.5% (w/v).

### Biomaterial Discs

Discs, 6 millimeters in diameter, were prepared by cutting ePTFE cardiovascular straight graft tubes (regular wall tubes, 8 millimeters in diameter Impra, Tempa, AT., USA). Discs were gradually dipped in the 5% (w/v) PVA solution. The discs were removed and excess solution was carefully drained. The coated discs were allowed to dry overnight at room temperature. Additional discs were prepared by independently dipping discs in PVA solutions of differing concentrations of 0.5, 1, and 2.5% (w/v).

### EXAMPLE 2

Biomaterials with a thromboresistant coating of a chitosan-PVA coating were prepared as follows.

### Preparation of PVA solution

A 5% (w/v) polymer solution was prepared by stirring 2.5 grams PVA (BDH, Cat. No. 2979) in 50 mLs of warm distilled water. Similar polymer solutions were prepared at concentrations of 0.5%, 1%, and 2.5% (w/v).

### Preparation of Chitosan Solution

A 3% (w/v) chitosan solution in 1% (v/v) acetic acid was prepared by suspending three grams of chitosan (70,000 MW Fluka CAT. No. 22741) in 99 mLs of water with magnetic stirring for 15 minutes. One mL of glacial acetic acid was then added and mixing continued for 16 hours at room temperature.

### Preparation of coating solution

A solution for the preparation of a chitosan-PVA coating containing 1.2% (w/v) chitosan and 0.6% (w/v) PVA was prepared by mixing 4 volumes of 3% chitosan solution with 1.2 volumes of 5% PVA solution. To this solution was added one volume of a 1% (v/v) Triton X-100 (BDH, CAT. No. 30632) and 3.8 volumes of 1% acetic acid.

A second solution for the preparation of a chitosan-PVA coating containing 0.6% (w/v) chitosan and 0.3% (w/v) PVA was prepared by mixing 2 volumes of 3% chitosan solution with 0.6 volumes of 5% PVA solution. To this solution was added 1 volume of a 1% (v/v) Triton X-100 (BDH, CAT. No. 30632) and 6.4 volumes of 1% acetic acid.

Solutions for the preparation of chitosan coatings containing 0.6% (w/v) chitosan and 1.2% (w/v) chitosan were prepared by mixing 4 or 2 volumes of 3% (w/v) chitosan solution with 5 or 7 volumes of 1.0% acetic acid, respecxtively, with 1 volume 1% (v/v) Triton X-100 (BDH, CAT. No. 30632).

### Biomaterial Discs

Discs, 6 millimeters in diameter, were prepared by cutting ePTFE cardiovascular straight graft tubes (regular wall tubes, 8 millimeters in diameter Impra, Tempa, AT., USA). Discs were gradually dipped, independently, in either of the chitosan-PVA solutions or chitosan solutions prepared above. The discs were removed and the excess solution was carefully drained. The coated discs were allowed to dry overnight at room temperature.

### EXAMPLE 3

Human blood platelets were isolated and labelled with ⁵¹Cr as follows:

### Platelet binding Assay

Venous blood was collected from healthy human volunteer using protocols approved by the Robert Wood Johnson Medical School Ethics Committee. Human platelets were isolated by gel filtration from freshly drawn blood anti-coagulated with acid-citrate dextrose (56 mM sodium citrate, 65 mM citric acid, 104 mM glucose) supplemented with prostaglandin E₁ (1 µM Sigma). Platelet-rich plasma was prepared by centrifugation of the blood at 180 x g for 20 minutes at room temperature. Platelets were pelleted from the platelet-rich plasma by centrifugation at 1000 x g for 16 minutes at room temperature. The pellet was gently resuspended in platelet-poor plasma. Prostaglandin E₁ (1µM) and apyrase (1 unit/mL Sigma) were added to each isolation step to minimize activation of the platelets. Platelets were isolated from the plasma by filtration through a Sepharose 2B column equilibrated with Walsh buffer (137 mM NaCl, 2.7 mM KCI, 1 mM MgCl₂, 3.3 mM NaH₂PO₄, 5.6 mM glucose, 20 mM HEPES, 0.1% bovine serum albumin (sigma), pH 7.4). The plasma free platelet count was adjusted to 2-3 x 10⁸ platelets/mL using Walsh solution. Platelet quantitation was done using a hemocytometer (Fisher Scientific).

To label platelets with ⁵¹Cr, 500 µCi of ⁵¹Cr (5 mCi/mL normal saline, ICN Biomedicals) was added to each mL of resuspended platelet solution in plasma. The resulting solution was incubated at 37°C for 60 minutes. The labeled platelets were filtered and diluted as stated above.

### TEST 1

### Platelet Adhesion Assay

The 6 millimeter coated discs of Example 1 were placed at the bottom of wells in 96-well microtiter plates (Corning). The surface of the discs were coated with platelet-poor plasma (100 µL/well) for one hour at room temperature. Non-adherent proteins were removed by aspiration and the wells were washed three times with phosphate buffered saline (PBS, 200 µL/well). ⁵¹Cr-labelled platelets (100 µL/well) (prepared in Example 4) were added to the wells for one hour. Non-adherent platelets were removed by aspiration and the wells were washed three times with phosphate buffered saline (PBS, 200 µL/well). Adhered platelets were lysed with lysis buffer (2% SDS, 66 mM Tris pH 7.4, 100 µL/well). The lysates were collected and ⁵¹Cr-labelled platelet adherence was quantitated using a gamma counter (LKB Wallac, Model 1282). Each condition was examined in triplicate. Platelet binding to untreated ePTFE discs was normalized in all experiments to 100%. Adhesion to the treated ePTFE surfaces is expressed as percentage binding relative to the binding to the untreated ePTFE surface. Results of different coatings are shown on the following table.

**Table I -**

| PVA Treatment of ePTFE Discs | |
|---|---|
| Treatment | % Platelet Binding |
| Control - No coating | 100 |
| Coated using a 0.5% PVA solution | 26 |
| Coated using a 1.0% PVA solution | 18 |
| Coated using a 2.5% PVA solution | 20 |
| Coated using a 5% PVA solution | 15 |

Treatment of the ePTFE discs with PVA at concentrations greater than about 1% reduced platelet binding to the surface by 80%.

### TEST 2

### Platelet Adhesion Assay

The 6 mm coated discs of Example 2 were placed at the bottom of wells in 96-well microtiter plates (Corning). The surface of the discs were coated with platelet-poor plasma (100 µL/well) for one hour at room temperature. Non-adherent proteins were removed by aspiration and the wells were washed three times with phosphate buffered saline (PBS, 200 µL/well). ⁵¹Cr-labelled platelets (100 µL/well) (prepared in Example 4) were added to the wells for one hour. Non-adherent platelets were removed by aspiration and the wells were washed three times with phosphate buffered saline (PBS, 200 µL/well). Adhered platelets were lysed with lysis buffer (2% SDS, 66 mM Tris pH 7.4, 100 µL/well). The lysates were collected and ⁵¹Cr-labelled platelet adherence was quantitated using a gamma counter (LKB Wallac, Model 1282). Each condition was examined in triplicate. Platelet binding to untreated ePTFE discs was normalized in all experiments to 100%. Adhesion to the treated ePTFE surfaces is expressed as percentage binding relative to the binding to the untreated ePTFE surface. Results of different coatings is shown on the following table.

**Table II -**

| PVA-Chitosan Treatment of ePTFE Discs | |
|---|---|
| Treatment | % Platelet Binding |
| Control - No coating | 100 |
| Coated using a 0.6% chitosan solution | 130 |
| Coated using a 1.2% chitosan solution | 105 |
| Coated using a 0.6% chitosan/0.3% PVA solution | 20 |
| Coated using a 1.2% chitosan/0.6% PVA solution | 15 |

Treatment of the ePTFE surface with the chitosan/PVA mixture reduced platelet binding by 80%. Treatment using only chitosan increased platelet binding to the ePTFE surface.

## Claims

1. A biomaterial having a thromboresistant surface, wherein said surface is comprised of a coating layer of chitosan and polyvinyl alcohol.

2. A medical device comprised of the biomaterial of claim 1.

3. The medical device of claim 2 wherein said device is a prosthetic vascular graft.

4. The graft of claim 3 wherein the graft is substantially tubular in cross-section.

5. The graft of claim 4 having an interior diameter of less than about 4 mm.

6. A surface treatment for biomaterials comprising the application of a coating solution comprising chitosan and polyvinyl alcohol to said biomaterial.

7. The surface treatment of claim 6 wherein said biomaterial is selected from the group consisting of polytetrafluoroethylenes, polyamides, polyesters, polyurethanes, polysiloxanes, polyolefins, Dacron, silicone rubber, polyvinyl chlorides, copolymers and metals.

8. The surface treatment of claim 6 wherein said coating is cured.

9. The surface treatment of claim 8 wherein said coating is cured by crosslinking.

10. The surface treatment of claim 9 wherein said biomaterial is Dacron or expanded polytetrafluoroethylene.

11. The surface treatment of claim 10 wherein the ratio of chitosan to polyvinylalcohol is in the range of about 9:1 to about 1:1.

12. The surface treatment of claim 6 wherein the application process comprises the steps of:
A) preparing a coating solution comprising a sterile aqueous solution of chitosan and polyvinyl alcohol;
B) coating the surface of the biomaterial with the coating solution; and,
C) curing the adsorbed solution.

13. The surface treatment of claim 12 wherein the coating solution further comprises additives such as, stabilizers, buffers, catalysts and cross-linking agents.

14. The surface treatment of claim 13 wherein the surface of the biomaterial is coated with the coating solution by pumping or by dipping.

15. The surface treatment of claim 14 wherein the adsorbed solution is cured either by drying or by chemical cross-linking, using a cross-linking agent in an aqueous solution.

16. The surface treatment of claim 15 wherein bioactive materials are entrapped within the adsorbed solution during the initial coating formation or bonded and immobilized to its surface at activated sites on the chitosan.

17. The surface treatment of claim 16 wherein the activated sites on the chitosan adsorbed solution are activated by glutardialdehyde.

## Patentansprüche

1. Ein Biomaterial mit einer thrombonenresistenten Oberfläche, **dadurch gekennzeichnet, dass** die schon erwähnte Oberfläche aus einer Beschichtung aus Chitosan und Polyvinylalkohol besteht.

2. Eine medizinische Einheit, die das Biomaterial gemäss Patentanspruch 1 beinhaltet.

3. Die medizinische Einheit gemäss Patentanspruch 2, **dadurch gekennzeichnet, dass** die schon erwähnte Einheit ein prosthetisches vaskulares Transplantat ist.

4. Das Transplantat aus Patentanspruch 3 ist im wesentlichen ein Transplantat mit tubulärem Querschnitt.

5. Das Transplantat aus Patentanspruch 4 hat einen Innendurchmesser von weniger als 4 mm.

6. Die Oberflächenbehandlung von Biomaterialien, die in dem Auftrag einer Beschichtungslösung aus Chitosan und Polyvinylalkohol auf das genannte Biomaterial besteht.

7. Die Oberflächenbehandlung gemäss Patentanspruch 6, wobei das erwähnte Biomaterial aus einer Gruppe aus Polytetrafluorethylenen, Polyamiden, Polyestern, Polyurethanen, Polysilikonen, Polyolefinen, Dacron, Silikonkautschuk, Polyvinylchloriden, Copolymeren und Metallen ausgesucht wird.

8. Die Oberflächenbehandlung gemäss Patentanspruch 6 , **dadurch gekennzeichnet, dass** die Beschichtung ausgehärtet wird.

9. Die Oberflächenbehandlung gemäss Patentanspruch 8, **dadurch gekennzeichnet, dass** die besagte Beschichtung durch Vernetzung ausgehärtet wird.

10. Die Oberflächenbehandlung gemäss Patentanspruch 9, **dadurch gekennzeichnet, dass** als Biomaterial Dacron oder aufgeschäumte Polytetrafluorethylene verwendet werden.

11. Die Oberflächenbehandlung gemäss Patentanspruch 10, bei der das Verhältnis von Chitosan zu Polyvinylalkohol in einem Bereich zwischen 9:1 und ungefähr 1:1 liegt.

12. Die Oberflächenbehandlung gemäss Patentanspruch 6 , wobei der Auftragungsprozess aus folgenden Schritten besteht:
A)Vorbereitung einer Beschichtungslösung, die aus einer sterilen Wasserlösung aus Chitosan und Polyvinylalkohol besteht;
B) Auftrag der Beschichtungslösung auf die Oberfläche des Biomaterials und
C) Aushärten der absorbierten Lösung.

13. Die Oberflächenbehandlung gemäss Patentanspruch 12, **dadurch gekennzeichnet, dass** die Beschichtungslösung zusätzliche Substanzen wie Stabilisatoren, Puffer, Katalysatoren und Vernetzungsagens enthält.

14. Die Oberflächenbehandlung gemäss Patentanspruch 13, **dadurch gekennzeichnet, dass** die Oberfläche des Biomaterials mit der Beschichtungslösung im Pump- oder Eintauchverfahren beschichtet wird.

15. Die Oberflächenbehandlung gemäss Patentanspruch 14, **dadurch gekennzeichnet, dass** die absorbierte Lösung entweder durch Trocknung oder durch chemische Vernetzung mittels eines Vernetzungsagens in einer wässerigen Lösung hergestellt wird.

16. Die Oberflächenbehandlung gemäss Patentanspruch 15, **dadurch gekennzeichnet, dass** bioaktive Materialien während der anfänglichen Schichtbildung in der absorbierten Lösung eingeschlossen oder an ihrer Oberfläche an aktivierten Punkten auf dem Chitosan gebunden und festgesetzt werden.

17. Die Oberflächenbehandlung gemäss Patentanspruch 16, wobei die aktivierten Punkte auf der Chitosan absorbierten Lösung mit Glutardialdehyd aktiviert werden.

## Revendications

1. Biomatériau ayant une surface thromborésistante, dans lequel ladite surface est constituée d'une couche d'enrobage de chitosane et d'alcool polyvinylique.

2. Dispositif médical composé par le biomatériau selon la revendication 1.

3. Dispositif médical selon la revendication 2, dans lequel ledit dispositif est un greffon vasculaire prothétique.

4. Greffon selon la revendication 3, dans lequel le greffon est substantiellement tubulaire en section transversale.

5. Greffon selon la revendication 4 ayant un diamètre intérieur inférieur à environ 4 mm.

6. Traitement de surface pour des biomatériaux comprenant l'application d'une solution d'enrobage comprenant du chitosane et de l'alcool polyvinylique sur ledit biomatériau.

7. Traitement de surface selon la revendication 6, dans lequel ledit biomatériau est choisi dans le groupe constitué par les polytétrafluoroéthylènes, les polyamides, les polyesters, les polyuréthanes, les polysiloxanes, les polyoléfines, le dacron, le caoutchouc de silicone, les polychlorures de vinyle, des copolymères et des métaux.

8. Traitement de surface selon la revendication 6, dans lequel ledit enrobage est vulcanisé.

9. Traitement de surface selon la revendication 8, dans lequel ledit enrobage est vulcanisé par réticulation.

10. Traitement de surface selon la revendication 9, dans lequel ledit biomatériau est du dacron ou du polytétrafluoroéthylène expansé.

11. Traitement de surface selon la revendication 10, dans lequel le rapport de chitosane à l'alcool polyvinylique se situe dans la plage d'environ 9/1 à environ 1/1.

12. Traitement de surface selon la revendication 6, dans lequel le procédé d'application comprend les étapes de :
A) préparation d'une solution d'enrobage comprenant une solution aqueuse stérile de chitosane et d'alcool polyvinylique ;
B) enrobage de la surface du biomatériau avec la solution d'enrobage ; et,
C) vulcanisation de la solution adsorbée.

13. Traitement de surface selon la revendication 12, dans lequel la solution d'enrobage comprend en outre des additifs tels que des stabilisants, des tampons, des catalyseurs et des agents de réticulation.

14. Traitement de surface selon la revendication 13, dans lequel la surface du biomatériau est enrobée avec la solution d'enrobage par pompage ou par immersion.

15. Traitement de surface selon la revendication 14, dans lequel la solution adsorbée est vulcanisée soit par séchage soit par réticulation chimique, en utilisant un agent de réticulation dans une solution aqueuse.

16. Traitement de surface selon la revendication 15, dans lequel les matériaux bioactifs sont encapsulés à l'intérieur de la solution adsorbée pendant la formation de l'enrobage initial ou liés et immobilisés sur sa surface aux niveaux de sites activés sur le chitosane.

17. Traitement de surface selon la revendication 16, dans lequel les sites activés sur la solution adsorbée de chitosane sont activés par du glutaraldéhyde.
